# EUROPÄISCHE PATENTSCHRIFT

(11) **EP 1 370 862 B1**
(45) Veröffentlichungstag und Bekanntmachung des Hinweises auf die Patenterteilung: **28.12.2005**
(21) Anmeldenummer: 02719978.5
(22) Anmeldetag: 28.02.2002
(51) Int. Cl.: G01N 33/00, G01N 27/00, G01N 29/02

(54) **METHODE UND VORRICHTUNG ZUM NACHWEIS VON METHYL-ISOTHIOCYANAT IN LUFTPROBEN**
METHOD AND APPARATUS FOR THE DETECTION OF METHYL ISOTHIOCYANATE IN AIR SAMPLES
PROCEDE ET DISPOSITIF POUR DETECTER LA PRESENCE DE METHYL-ISOTHYOCYANATE DANS DES ECHANTILLONS D'AIR

(30) Priorität: 28.02.2001 DE 10109534
(43) Veröffentlichungstag der Anmeldung: 17.12.2003
(73) Patentinhaber: BASF Aktiengesellschaft, 67056 Ludwigshafen (DE)
(72) Erfinder: KOBER, Reiner, 67136 Fussgönheim (DE); STEGMAIER, Wolf, 67459 Böhl-Iggelheim (DE); ZIEGLER, Hans, 67112 Mutterstadt (DE); PROBECK, Erich, 67278 Bockenheim (DE); CHRISTEN, Thomas, 67125 Dannstadt-Schauernheim (DE); BARGON, Joachim, 53177 Bonn (DE); VÖGTLE, Fritz, 53347 Alfter-Impekoven (DE); HORNER, Gerhard, 82515 Wolfratshausen (DE)
(74) Vertreter: Kinzebach, Werner
(86) Internationale Anmeldenummer: PCT/EP2002/002206
(87) Internationale Veröffentlichungsnummer: WO 2002/068953

(56) Entgegenhaltungen:
- WO-A-00/78204
- GB-A- 590 368
- GB-A- 2 165 948
- US-A- 5 332 580
- US-A- 5 469 369
- US-A- 5 639 956
- US-A- 6 085 576
- STETTER J R ET AL: "New sensor arrays and sampling systems for a modular electronic nose" SENSORS AND ACTUATORS B, ELSEVIER SEQUOIA S.A., LAUSANNE, CH, Bd. 69, Nr. 3, 25. Oktober 2000 (2000-10-25), Seiten 410-419, XP004218245 ISSN: 0925-4005
- SZETO S Y ET AL: "Nonpoint-source groundwater contamination by 1,2,2-trichloropropane, a trace impurity in soil fumigant formulations" J ENVIRON QUAL;JOURNAL OF ENVIRONMENTAL QUALITY NOV-DEC 1994 AMERICAN SOC OF AGRONOMY INC, MADISON, WI, USA, Bd. 23, Nr. 6, November 1994 (1994-11), Seiten 1367-1370, XP002206312
- PATENT ABSTRACTS OF JAPAN vol. 2000, no. 19, 5. Juni 2001 (2001-06-05) & JP 2001 031510 A (NIPPON KUNJO GIJUTSU KYOKAI), 6. Februar 2001 (2001-02-06)
- LI D ET AL: "Surface acoustic wave microsensors based on cyclodextrin coatings" SENSORS AND ACTUATORS B, ELSEVIER SEQUOIA S.A., LAUSANNE, CH, Bd. 69, Nr. 1-2, 10. September 2000 (2000-09-10), Seiten 75-84, XP004208562 ISSN: 0925-4005
- SOUTEYRAND E ET AL: "Behaviour Of Cryptophane Molecules In Gas Media" TRANSDUCERS95, EUROSENSORS IX, 25. - 29. Juni 1995, Seiten 882-885, XP010304938 stockholm
- HIERLEMANN A ET AL: "Polymer-based sensor arrays and multicomponent analysis for the detection of hazardous organic vapors in the environment" SENSORS AND ACTUATORS B, Bd. 26-27, 1995, Seiten 126-134, XP002204810 elsevier
- DALCANALE E ET AL: "Selective detection of organic compounds by means of cavitand-coated QCM transducers" SENSORS AND ACTUATORS B, ELSEVIER SEQUOIA S.A., LAUSANNE, CH, Bd. 24, Nr. 1-3, März 1995 (1995-03), Seiten 39-42, XP004302098 ISSN: 0925-4005
- HEIL C. ET AL: 'HIGHLY SENSITIVE SENSOR MATERIALS FOR DISCRIMINATING CARBONYL COMPOUNDS IN THE GAS PHASE USING QUARTZ MICROBALANCES' SENSORS AND ACTUATORS B Bd. 61, 1999, ELSEVIER, Seiten 51 - 58

## Beschreibung

Die vorliegende Erfindung betrifft ein Verfahren und Vorrichtung zum Nachweis von MITC in Luftproben.

Die in Landwirtschaft oder in Gärtnerei genutzten Böden können durch pflanzenschädigende Organismen, sogenannte Phytopathogene, wie Nematoden, im Boden lebende Insekten, keimende Pflanzen, Bodenbakterien oder Bodenpilze befallen sein. Beispielsweise schätzt man den durch den Befall von Nutzpflanzen durch Boden- oder Wurzelnematoden resultierenden Ernteverlust weltweit auf ca. 12%, was Einnahmeverlusten der Erzeuger in Höhe mindestens 7 Milliarden US Dollar entspricht. Außerdem erfordern die Bestimmungen der internationalen Pflanzenquarantäne, dass nicht nur das exportierte und importierte Pflanzenmaterial frei von Nematoden ist, sondern dass es auch in nematodenfreien Böden gewachsen ist. Es ist daher häufig erforderlich, landwirtschaftlich genutzte Böden vor der nächsten Aussaat oder Wiederanpflanzung zu entseuchen, beispielsweise durch Behandlung mit einem Fungizid oder einem Nematizid.

Zur Bodenentseuchung werden meist sogenannte Fumigantien (Räucher- oder Bodenbegasungsmittel) eingesetzt. Fumigantien werden üblicherweise in flüssiger Form oder in fester Form appliziert. Während flüssige Formulierungen im Boden aufgrund ihres hohen Dampfdruckes wirken, zerfallen feste Verbindungen, die beispielsweise in Form eines Granulats in den Boden eingebracht werden, unter dem Einfluß der Bodenfeuchte in gasförmige, biozidwirksame Verbindungen. Die Präparate diffundieren durch das Kapillarsystem des Bodens, wo sie dann die Schädlinge als Atemgift treffen. Bei direktem Kontakt können Fumigantien auch als Kontaktgift wirken.

In den vergangenen Jahrzehnten war Methylbromid das am weitesteren verbreitete Bodenbegasungsmittel. Methylbromid ist aber bekanntlich eine Substanz, die zur Schädigug der Ozonschicht der Erde beiträgt. Daher haben über 100 Staaten 1997 in einer Folgekonferenz zum "Montreal Protocol on Substances that Deplete the Ozon Layer" beschlossen, dass Methylbromid ab dem Jahre 2005 in Industriestaaten und ab dem Jahre 2015 in Entwicklungsländern nicht mehr eingesetzt werden darf. 1998 hat der US-Kongress ein bereits für das Jahr 2001 vorgesehenes Verbot von Methylbromid in Anpassung an das "Montreal Protocol" bis ins Jahr 2005 verschoben.

Anstelle von Methylbromid werden daher vermehrt andere Bodenentseuchungsmittel in Betracht gezogen.

So beschreibt die US-Patentschrift US-A 2,838,389 die Verwendung von Tetrahydro-3,5-dimethyl-1,3,5-thiadiazin-2-thion nach der Formel 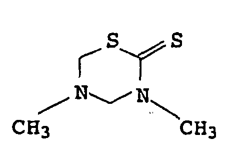 mit der Kurzbezeichnung "Dazomet" als Mittel zur Bodenentseuchung in der Landwirtschaft und Gärtnerei. Bei der Anwendung von Dazomet wird Methyl-iso-thio-cyanat (MITC) mit der Formel

Me - N = C = S

als eigentliches biologisch wirksames Agens freigesetzt. Dazomet selbst stellt daher nur eine sogenannten Prodrug-Vorstufe dar. In der neueren Literatur wird die Bodenentseuchung mit Dazomet in Form von Basamid®-Granulaten (Basamid ist eine eingetragene Marke der BASF AG, Ludwigshafen) beschrieben in Forest *Prod. J. 43 (2) (1993), S. 41-44;* in Acta *Horticulture 382 (1995) S. 110* ff und in *Fand Fiber Science* 27*(2) (1995), S. 183-197.*

Basamid®-Granulat ist eine relativ inerte feste Substanz, die erst bei Ausbringung auf den feuchten Boden aktiv wird. Bevorzugte Anwendungsgebiete für Basamid® sind der Zierpflanzen-, Gemüse- und Tabakanbau, Baumschulen, der Obst-, Wein- und Hopfenanbau sowie die Entseuchung von Kompost- und Gewächshauserde. Das 98 bis 100% Dazomet enthaltende Basamid®-Granulat kommt als Streumittel zur Anwendung und wird 20 bis 30 cm tief, je nach Anforderung auch bis zu 50 cm tief in den Boden eingearbeitet. Anschließend wird der Boden üblicherweise mit einer Plastikfolie abgedeckt, um ihn während der Behandlungszeit feucht zu halten und um die wirksame gasförmige Komponente nicht zu rasch zu verlieren.

Ein weiteres flüssiges Bodenentseuchungsmittel, welches ebenso wie Dazomet MITC freisetzt, ist Natrium-methylcarbamodithionat, welches unter den Handelsnamen VAPAM, METHAMFLUID, AAMONAN oder DICID vertrieben wird. Ebenso wird vielfach MITC direkt oder formuliert unter den Handelsnamen TRAPEX und VORAX zur Begasung eingesetzt. Es kann auch als Gemisch mit Metham verwendet werden. Das Mittel kommt typischerweise als 20%ige Lösung in einem organischen Lösungsmittel zur Anwendung, das die Flüchtigkeit der Wirksubstanz unterstützen soll.

Ein weiteres flüssiges Bodenentseuchungsmittel und Nematizid ist 1,3-Dichlorpropen(1,3 DCP), das unter den Handelsnahmen DCP 50, SCHELL-DD, TELONE oder DI-TRAPEX vertrieben und gegebenenfalls auch in Kombination mit MITC eingesetzt wird.

Die bekannten Bodenbegasungsmittel besitzen eine hohe Phytotoxizität. Sie können daher nur dort eingesetzt werden, wo die zu behandelnden Flächen von den Nutzkulturen geräumt sind. Nach jeder Anwendung eines Bodenentseuchungsmittels ist daher eine gewisse Wartezeit bis zur Wiederaussaat bzw. zur Wiederanpflanzung von Nutz- und Kulturpflanzen einzuhalten, um sicherzustellen, dass das Bodenentseuchungsmittel so weit abgebaut ist, dass keine nachteiligen Auswirkungen auf die Wiederaussaat bzw. Wiederanpflanzung zu befürchten sind.

Über Aktivierung, Freisetzung und Abbau der biologisch wirksamen Substanzen der obengenannten Bodenbegasungsmittel ist jedoch noch vergleichsweise wenig bekannt. Wichtige Einflußgrößen, die den Metabolismus der Bodenbegasungsmittel im Boden beeinflussen, sind aber die Temperatur, der Wassergehalt und pH-Wert des Bodens. Auch der Bodentyp, beispielsweise das Vorhandensein von Übergangsmetallen, spielt hier eine gewisse Rolle.

Ebenso hat die Verabreichung von Düngemitteln und anderen Stoffen einen wesentlichen Einfluß auf die Geschwindigkeit, mit der Bodenbegasungsmittel aus dem Boden freigesetzt werden. So wurde erst kürzlich gezeigt, dass sich die normalerweise hohe Flüchtigkeit von 1,3-DCP durch Düngemittel, die Thiosulfate enthalten, reduziert (Gan et al, *Journal* of *Environmental Quality 29(5) (2000), Seiten 1476-1481).*

Aufgrund dieser vielfältigen Einflußgrößen kann bei einer konkreten Anwendung der zeitliche Verlauf der MITC- oder 1,3-DCP-Konzentration im Boden ohne Messungen nur völlig unzureichend abgeschätzt werden.

Jedoch nicht nur die Kenntnis des genauen Restgehaltes von MITC und 1,3 DCP im Boden ist wegen der Phytotoxizität dieser Substanzen für den Anwender von Bedeutung. Bereits bei der Anwendung ist aufgrund der toxischen Wirkung dieser Substanzen eine Überwachung der Raum- oder Umgebungsluft aus Gründen der Arbeitssicherheit notwendig. So hat sich beim Einsatz von Dazomet-Granulat gezeigt, dass bei hohen Bodentemperaturen über 30 °C und ausreichender Bodenfeuchte die Freisetzung von MITC sehr schnell erfolgt. Insbesondere im Fall von Gewächshausanwendungen hat man beim Einarbeiten des Granulats in den Boden beobachtet, dass das freigesetzte MITC bei nicht sachgemäßer Lüftung und Anwendung zu temporären Schleimhaut- und Augenreizungen führen kann. Ferner ist in diesem Zusammenhang bekannt, dass Metam-Fluid bzw. Metam-Natrium aufgrund der sehr schnellen und hohen Gasentwicklungstendenz im Gewächshaus nur bedingt einsatzfähig ist oder, wie z.B. in Kalifornien, für derartige Anwendungen überhaupt nicht mehr zugelassen ist. Ohne ein schnelles und zuverlässiges Messverfahren besteht daher die Gefahr, dass Landarbeiter und andere Anwender dem freigesetzten MITC oder 1,3-DCP unnötig lange bzw. in einer zu hohen Konzentration dieser Gase ausgesetzt werden.

Als analytische bzw. chromatographische Verfahren sind zum Nachweis der Bodengase MITC und/oder 1,3-Dichlorpropen verschiedene HPLC- und GC-Methoden bekannt (vergl. z.B. Subramanian, *Environm. Toxicol: Chem. 15 (1996), Seiten 503-513). So* erfolgte z.B. bisher der Nachweis der Exposition von Landarbeitern beim Kartoffelaribau gegenüber dem Nematozid cis-1,3-Dichlarpropen (cis-DCP) und die Bestimmung von dessen wirkung auf den Menschen durch eine urinuntersuchungen. In einer holländischen Studie wurde erst kürzlich festgestellt, dass an mehr als 20% der Tage im Beobachtungszeitraum die gesetzlich festgelegte Obergrenze der täglichen Exposition überschritten wurde (Brouwer et al., *Occupational and Environmental* Medicine, *57 (11) (2000),* Seiten *738 -744).*

Ebenso sind chemische, nass-chemische organische und anorganische .. Nachweismethoden von Isothiocyanten bekannt. So offenbart Bull. Chem. Soc. Jap. 52, *(1979),* Seiten *2155-2156,* den Nachweis von Isothiocyanaten mit Iodmonochlorid über die Bildung von Alkylthioharnstoffen durch die Umsetzung der Isothiocyanate mit Alkylamin.

Diese Nachweismethoden sind aber entweder zu unempfindlich oder so kompliziert, dass sie nur in gut eingerichteten Labors durchgeführt werden können. Für den alltäglichen Einsatz beim Anwender von Pflanzenschutzmitteln sind diese Methoden daher nicht geeignet.

Für den Anwender ist es aber insbesondere wichtig, nach erfolgter Applikation von Metam-Natrium, 1,3-Dichlorpropen und/oder Dazomet zu prüfen, ob sich noch Restmengen an MITC und/oder 1,3-DCP im Boden befinden, die noch nicht mineralisiert bzw, abgebaut sind. Falls dies der Fall ist, muss der Anwender noch eine gewisse Wartezeit einhalten, da Restmengen dieser Substanzen im Boden zu Pflanzenschäden bei der Wiedereinsaat bzw. der Wiederanpflanzung von Nutz- und Kulturpflanzen können. Insbesondere bei Verwendung des Feststoff-Produktes Dazomet kommt dem MITC-Nachweis eine besondere Bedeutung zu, da, wie oben bereits beschrieben, die Freisetzung und Mineralisierung von MITC stark von Bodenbeschaffenheit, Temperatur, Feuchtigkeit, Düngemittelart und -konzentration u. a. Gegebenheiten beeinflußt wird.

Üblicherweise verwenden Anwender bislang ein speziell für den Einsatz vor Ort entwickeltes biologisches Testsystem mit Kresse- bzw. Tabaksamen. Es beruht darauf, dass diesse Samen durch geringste Mengen an MITC keimungsinhibiert werden und dadurch indirekt Restmengen von MITC anzeigen. Dieser sogenannte "Kresse-Test" ist allerdings umständlich und verlangt seinerseits eine mehrtägige Wartezeit, bis gezeigt werden kann, dass z. B. die Kresse aufläuft, also anwächst. Ein weiterer Nachteil derartiger biologischer Test besteht darin, dass MITC beispielsweise durch fehlerhaftes Wassermanagement falsch positiv bzw. falsch negativ nachgewiesen bzw. angezeigt wird.

Aus der internationalen Patentanmeldung WO 99/66304 ist ein Verfahren und eine Vorrichtung zur Detektion von Spuren eines Analyten mittels eines künstlichen Riechsystems bekannt. Während sich diese Anmeldung in erster Linie mit medizinischen Anwendungen wie dem Nachweis von Halitosis oder der Periodontalerkrankung beschäftigt, werden zahlreiche andere Anwendungsbereiche, unter anderem der Nachweis von Fumigantien, sowie zahlreiche unterschiedliche Sensortypen kurz erwähnt. Abgesehen von dem Vorschlag die zu untersuchenden Fluide aufzukonzentrieren, beschreibt WO 99/66304 keine technische Lehre wie Analytspuren in Luftproben zuverlässig nachgewiesen werden können. Insbesondere beschäftigt sich dieses Dokument nicht mit dem Nachweis von MITC oder 1,3-DCP.

US-A-5639956 offenbart eine Vorrichtung zum elektrochemischen Nachweis von chlorierten Kohlenwasserstoffen und Permanentgasen in Bodenluftproben. Für die selektive Detektion von chlorierten Kohlenwasserstoffen und toxischen aromatischen Verbindungen in Anwesenheit von Permanentgasen sind schon mit Cavitanden beschichtete Quarzmikrowaagen entwickelt worden *(Dalcanale et al., Sensors and Actuators B,* 24-25 (1995) 39-42).

Heil et al. untersuchten auf Dendrimer und/oder Makrozyklen basierende Sensormaterialien zum Nachweis von Carbonylverbindungen, die auf Quarzmikrowaagen aufgebracht waren *(Heil et al., Sensors and Actuators B, 61 (1999) 51-58*).

Für die sensorische Erfassung von Methylisothiocyanat (MITC) auch schon ein Biosensor entwickelt worden (Iwuoha et al. *Analytical Chemistry, 69(8) 1674-1681 (1997).* Dieser Biosensor benutzt amperometrische Enzym-Elektroden, die in flüssigen, organischen Phasen arbeiten (OPEEs = organic phase enzyme electrodes). Dank deren Verfügbarkeit können solche Verbindungen, die als Substrate für Oxidoreductasen qualifizieren, in einem geeigneten Lösungsmittel, d.h. in flüssiger Phase, erfasst werden, ohne dass eine umfassende Probenaufbereitung notwendig wird. Vor der Verfügbarkeit von OPEEs konnten nur solche Analyte erfaßt werden, die in Wasser löslich sind. Allerdings sind die von den OPEEs gelieferten Sensorsignale wesentlich schwächer als die Sensorsignale, die Elektroden in Wasser liefern, die mit natürlichen Enzymen belegten sind. Nach Iwuoha et al. (supra) kann man die Empfindlichkeit der OPEEs auf zwei Arten verbessern: Entweder man arbeitet mit natürlichen Enzymen in einem Lösemittelgemisch bestehend aus einem organischen Medium (z.B. Acetonitril, CH₃CN) und Wasser, oder man modifiziert die natürlichen Enzyme durch Veränderung von deren Aminosäurebausteine, d. h., man verwendet künstliche Enzyme. Die beiden von Iwuoha et al. beschriebenen Biosensor-Typen verwenden alternativ jeweils eines der beiden Prinzipien. Als Basisenzym dient universell die horseradish peroxidase (HRP). Diese reagiert mit H₂O₂ und ergibt ein Sensorsignal. Da MITC aber HRP inhibiert, ändert eine vorhandene Konzentration von MITC das Ansprechverhalten des entsprechend beschichteten Biosensors gegenüber einer bestimmten Konzentration von H₂O₂. Als hierfür angepasst modifizierte Enzyme kommen zum Einsatz entweder eine HRP, die mit dem homobifunktionellen Agens Suberinsäure-bis(N-hydroxybern-steinsäureimidester) zu SA-NHS modifiziert ist, bzw. ein mit Ethylenglycol-bis-(N-Hydroxy-bernsteinsäureimidester) zu EG-NHS modifiziertes Enzym. Die Elektroden wurden mit Hilfe einer in der Literatur beschriebenen elektrostatischen Komplexierungstechnik hergestellt.

Der bekannte Biosensor für MITC ist aber für den praktischen Einsatz in der Landwirtschaft und der Gärtnerei nicht geeignet, da der gasförmige Analyt zunächst in einem geeigneten Lösungsmittel gelöst und dann in gelöster Form mit dem eigentlichen Sensor in Kontakt gebracht werden muß. Ein solcher Sensor ist wegen der erforderlichen Behälter für frische und gebrauchte Lösungsmittel, für Spülflüssigkeiten und Spülgase und der entsprechend aufwendigen Leitungsanordnung nicht nur teuer in der Herstellung, sondern. besitzt auch ein hohes Gewicht und ist in der Handhabung sehr kompliziert und anfällig, so dass er sich für ein mobiles Analysegerät nicht eignet. Außerdem ist die erreichbare Nachweisempfindlichkeit für den Einsatz in der Landwirtschaft und in der Gärtnerei zu gering.

Der vorliegenden Erfindung liegt daher das technische Problem zugrunde ein einfaches und zuverlässiges Verfahren zum Nachweis von MITC in Luftproben bereitzustellen, das vor Ort vom Anwender von Bodenentseuchungsmitteln, sei es in der Landwirtschaft oder sei es in Gärtnereien, eingesetzt werden kann und das empfindlich genug ist, Restmengen von Fumigantien in Bodenluft oder Umgebungsluft sicher und genau nachzuweisen. Mit dem erfindungsgemäßen Verfahren sollen insbesondere Risiken bei der Wiedereinsaat bzw. der Wiederanpflanzung von Nutz- und Kulturpflanzen, beispielsweise nach Basamid- Applikation vermieden werden, ohne dass der Anwender auf den bislang üblichen, umständlichen und zeitaufwendigen Kresse-Test zurückgreifen muß. Es soll auch ein Verfahren zur Bekämpfung von Phytopathogenen mit Bodenentseuchungsmitteln bereitgestellt werden, das einen zuverlässigeren und kontrollierteren Einsatz der Bodenentseuchungsmittel erlaubt. Schließlich soll eine preiswerte und kompakte Vorrichtung zum Nachweis von MITC in Luftproben bereitgestellt werden, die ohne analytisch chemische Kenntnisse benutzt werden kann und die als leichtes und kompaktes tragbares Analysegerät eingesetzt werden kann.

Zur Lösung dieses technischen Problems schlägt die Erfindung vor, MITC in Luftproben mittels Chemosensoren zu messen. Chemosensoren sind Messfühler, die eine für eine chemische Substanz spezifische Meßgröße in auswertbares Signal, insbesondere ein elektrisches Signal umsetzen. Derartige Sensoren können auf unterschiedlichen physikalischen Messprinzipien beruhen. Als sensorempfindliches Element kann beispielsweise ein Halbleiterbauelement, wie etwa ein "Metal Oxide Semi-Conductor" (MOS) oder ein "Metal Oxide Semi-Conductor Field Effect Transistor" (MOSFET), dienen, aber auch elektrisch leitende Polymere, die als sogenannte "conducting polymer sensors" (CPS) bekannt sind. Überraschend wurde nun gefunden, dass massenempfindliche Sensoren, die mit einer für das nachzuweisende MITC selektiven Oberflächenschicht versehen sind, ganz besonders geeignet sind, MITC in Luftproben mit der zum Einsatz in der Landwirtschaft und in Gärtriereien erforderlichen Empfindlichkeit nachzuweisen.

Massenempfindliche. Sensoren sind beispielsweise als sogenannten Schwingquarzwagen "quarz micro balance" (QMB) oder als oberflächenwellenresonatoren, "surface acoustic wave devices" (SAW) bekannt. Schwingquarzwagen werden beispielsweise in Beschichtungsanlagen, etwa in Sputteranlagen, zur Kontrolle der Beschichtungsdicke eingesetzt. Üblicherweise wird ein Quarzoszillator in einen elektrischen Schwingkreis integriert. Der Quarzkristall wird mit metallischen Elektroden kontaktiert und unter Ausnutzung des umgekehrten piezoelektrischen Effektes mit einer typischerweise im Radiofrequenzbereich liegenden Frequenz angeregt, die einer mechanischen Eigenfrequenz des Quarzes entspricht. Es kommt dann zur Anregung von Resonanzschwingungen, die eine stabile Schwingungsfrequenz des Schwingkreises festlegen. Die Resonanzfrequenz hängt nun von der Masse des Quarzoszillators ab, so dass Masseänderungen, beispielsweise durch Adsorption oder Absorption einer nachzuweisenden Substanz, als Änderungen der Resonanzfrequenz detektiert werden können. Durch elektrische Brückenschaltungen lassen sich Frequenzänderungen im Bereich von 1 Hz messen.

Gegenstand der vorliegenden Erfindung ist daher ein Verfahren zum Nachweis von MITC in Luftproben, gemäß Anspruch 1.

Erfindungsgemäß kann man MITC beispielsweise in Bodenluft aus Erdböden, die mit Bodenentseuchungsmitteln behandelt wurden, nachweisen. Zur Überwachung der Exposition von Landarbeitern oder von Arbeitern in Gewächshäusern kann man den massenempfindlichen Sensor auch zum Nachweis von MITC in bodennaher Luft, bevorzugt bis zu einer Höhe von 1 bis 3 m über dem Erdboden oder in Raumluft, insbesondere in Gewächshäusern, verwenden.

Besonders bevorzugt wird der massenempfindliche Sensor zum Nachweis von MITC verwendet. Der massenempfindliche Sensor kann aber auch zum Nachweis anderer gasförmigen Fumigantien, insbesondere zum Nachweis von Methylbromid und/oder 1,3-dichlorpropen (1,3-DCP) verwendet werden.

Der Sensor weist eine Beschichtung auf, die für die nachzuweisende Substanz MITC möglichst selektiv ist. Im Idealfall würde daher ein einzelher Sensor mit einer hochspezifischen Beschichtung ausreichen, um die gesuchte Substanz nachzuweisen. Luftproben, insbesondere Bodenluft aus dem landwirtschaftlichen Bereich weist aber eine Vielzahl unterschiedlicher Substanzen auf. Neben den in der atmosphärischen Luft vorhandenen Gasen treten in der Bodenluft, vor allem infolge des durch MikroOrganismen bedingten Abbaus zersetzbarer organischer Substanzen, erhöhte Gehalte an CO2 auf (typischerweise 0,3 - bis 3,0 vereinzelt aber auch bis zu 10 Vol-%). Daneben werden auch noch andere Gase in Böden vor allem durch mikrobielle Prozesse gebildet. Je nach Stoffbestand und den jahreszeitlich unterschiedlichen Eh-pH-Bedingungen der verschiedenen Böden sind dies beispielsweise N₂O, NO, NO₂, NH₃, SO₂, H₂S, CH₄, C₂H₄, sowie weitere Substanzen mit relativ hohem Dampfdruck. Außerdem muss je nach Ausmaß der Belastung der Umgebungsluft und der Böden mit der Anwesenheit flüchtiger organischer Verbindungen wie Kraftstoffen, Lösemitteln u.a. aus anthropogenen Quellen in der Bodenluft gerechnet werden. So wurden selbst in weitgehend unbelasteten Böden Süddeutschlands vor ca. 15 Jahren Gehalte an Tetrachlorethylen in der Bodenluft von 0,1 bis 112 µg/m3 und ebenso erhöhte Gehalte an Trichlorethen und Trichlorethan gemessen.

Beschichtete massenempfindliche Sensoren weisen üblicherweise eine mehr oder wenige große Empfindlichkeit für einzelne, normalerweise aber für mehrere Komponenten eines Gasgemisches auf. Häufig sprechen Chemosensoren auch durch sogenannte "Querempfindlichkeiten" auf artverwandte Stoffe ähnlich sensibel an. Daher sind zur Unterscheidung bzw. zum eindeutigen Nachweis selbst nur einer einzigen chemischen Verbindung meistens mehrere Sensoren in geeigneten Kombinationen, sogenannten Sensorarrays, notwendig. Entsprechende Systeme, die auf unterschiedlichsten chemosensorischen Messprinzipien beruhen, sind für andere Anwendungszwecke als den Nachweis von Fumigantien bereits in der Literatur als sogenannte "elektronische Nasen" beschrieben worden.

Erfindungsgemäß wird man daher bevorzugt mehrere Sensoren verwenden, die vorzugsweise mit unterschiedlichen selektiven Schichten beschichtet sind. Grundsätzlich wird man um so mehr Sensoren benötigen, je unspezifischer die Beschichtungen der einzelnen Sensoren für die nachzuweisenden Substanzen sind und je breiter der Anwendungsbereich des Sensorarrays sein soll. zum Nachweis von MITC, werden vorzugsweise zwei bis zwölrf, insbesondere etwa sechs unterschiedliche Sensoren eingesetzt.

Die bisher in der Literatur beschriebenen selektiven Beschichtungen sind aber für den Nachweis von Fumigantien in Luftproben entweder ungeeignet oder zu unempfindlich. Auswahlkriterien für die Selektion und Kombination geeigneter Schichten beschreiben z.B.: Nakamura et al., *Sensors and Actuators B*. *69 (3)*, *295-301(2000).* Ferner wurden piezoelektrische Sensorsysteme mit Gold-Elektroden und AT-geschnittenen Quarz-Kristallen beschrieben, die mit Polydimethylsiloxan, Polyetherurethan, Polyethylcellulose bzw. Polycyanopropylmethylsiloxan beschichtet und als "array" bestehend aus 4 piezoelektrischen Sensoren betrieben werden. Die Quarze befanden sich dabei in einer thermostatisierbaren Kammer, die mit einer Gaszufuhr und einem -auslaß ausgestattet war. Sowohl die Datenerfassung wie auch die Gasmischung und deren Durchfluß wurden von einem Personalcomputer gesteuert. Die Eichung der Konzentrationen der Gase in der Kammer bzw. der Empfindlichkeit der Sensoren wurde durch isotherme Exposition bei zimmertemperatur vorgenommen. Die Konzentrationen der Gase beliefen sich auf 100 bis 1000 ppm für Toluen und Chloroform, bzw. auf 250 bis 2000 ppm für n-Octan.

Es wurde nun überraschend gefunden, dass ein Beschichtung des massenempfindlichen Sensors mit Makrozyklen und/oder Dendrimeren eine besonders geeignete selektive Beschichtung für den Nachweis von MITC darstellt. Derartige Beschichtungen wurden beispielsweise schon für den gravimetrischen Nachweis von Lösungsmitteldämpfen in Ehlen et al., Angew. *Chem., Int. Ed. English 32, 111-112 (1993)* beschrieben. Weiterhin wurden derartige selektive Beschichtungen zum Nachweis von Carbonylverbindungen in der Gasphase, und von Ammoniak verwendet.

Das bevorzugte Einsatzgebiet des erfindungsgemäßen Sensors liegt im Bereich der Landwirtschaft, wo er auch von messtechnisch weniger versierten Personen benutzt werden soll. Es ist daher wünschenswert, dass der massenempfindliche Sensor besonders robust, einfach zu handhaben und preiswert ist. Um dennoch hochempfindliche Messungen durchführen zu können, wird man gemäß einer besonders bevorzugten Ausführungsform des erfindungsgemäßen Verfahrens die nachzuweisenden Fumigantien zunächst anreichern, bevor man sie mit dem massenempfindlichen Sensor in Kontakt bringt. Dazu kann man beispielsweise das Bodengas zunächst durch einen Ad- oder Absorber, beispielsweise aus Kieselgel oder "TENAX" leiten oder aber es wird der Analyt zunächst kondensiert und anschließend mit Hilfe eines Inertgases (beispielsweise Luft oder Stickstoff) in konzentrierter Form dem Sensor zugeführt.

Vorteilhaft wird außerdem der Feuchtigkeitsgehalt der Luftprobe bestimmt, so dass man beispielsweise bei zu trockener Luft Hinweise auf eine unzureichende Umsetzung von Dazomet im Boden erhält. Bei zu trockener Luft kann man beispielsweise durch ein Signal anzeigen, dass der gemessene Wert unzuverlässig sein kann, weil möglicherweise noch weiteres nicht umgesetztes Dazomet im Boden enthalten ist.

Zur Messung von Bodenluftproben kann man beispielsweise eine Sonde einige Zentimeter bis einige zehn Zentimeter tief in den Boden einstechen und eine bestimmte Menge Luft mittels einer Saugpumpe über die Sonde ansaugen und an dem massenempfindlichen Sensor vorbeileiten. Die nachzuweisende Substanz lagert sich an dem massenempfindlichen Sensor ab und verändert die Resonanzfrequenz eines Schwingkreises, in welchen der Sensor integriert ist.

Gemäß einer anderen Ausführungsform der Erfindung wird eine zu prüfende Bodenprobe, beispielsweise einige 100 g, in ein Gefäß gefüllt. Anschließend wird Luft aus dem Gefäß mit den aus der Bodenprobe freigesetzten Fumigantien in eine Messkammer geleitet, in der das Sensorarray angeordnet ist.

Zur Erhöhung der Messgenauigkeit kann man der Luftprobe vor der Einleitung in die Messkammer Feuchtigkeit entziehen.

Die vorliegende Erfindung betrifft auch ein Verfahren zur Bekämpfung von Phytopathogenen, welches dadurch gekennzeichnet ist, dass man Erdboden mit einer wirksamen Menge eines Bodenbegasungsmittels behandelt und anschließend freigesetzte fumigantien mit dem oben beschriebenen Verfahren unter Verwendung eines massenempfindlichen Sensors nachweist.

Gegenstand der vorliegenden Erfindung ist außerdem eine Vorrichtung zum Nachweis von MITC in Luftproben gemäß Anspruch 7.

Als massenempfindlicher Sensor wird wenigstens eine Schwingguarzwaage eingesetzt. Der Schwingquarz stellt einen piezoelektrischen Resonator in einem elektrischen Schwingkreis dar. Änderungen der Massenbelegung des Resonators führen zu einer Verschiebung der Resonanzfrequenz des Schwingkreises, die elektronisch ausgewertet werden kann.

Bevorzugt verwendet man ein Array von massenempfindlichen Sensoren, von denen zumindest ein Sensor mit einer für das nachzuweisende MITC selektiven Oberflächenschicht beschichtet ist. Vorzugsweise beschichtet man aber mehrere Sensoren mit unterschiedlichen selektiven Schichten und wertet die erhaltenen Signale mittels sogenannter chemometrischer Methoden aus.

Die für das nachzuweisende MITC selektive Oberflächenschicht umfasst bevorzugt Makrozyklen, beispielsweise vom Lactam-Amid-Typ, und/oder Dendrimere. Es wurde gefunden, dass durch geeigneten molekularen Aufbau derartiger Makrozyklen und/oder Dendrimere eine hohe Selektivität der Schicht für die Einlagerung/Adsorption von MITC erreicht werden kann.

Gemäß einer bevorzugten Variante der erfindungsgemäßen Vorrichtung ist außerdem wenigstens eine Anreicherungseinheit für das nachzuweisende MITC vorgesehen.

Vorzugsweise ist im Strömungsweg stromaufwärts von der Messkammer eine Dampfsperre vorgesehen, welche für die nachzuweisenden Fumigantien permeabel und für die in der Luftprobe enthaltene Feuchtigkeit impermeabel ist. Bevorzugt umfasst die Dampfsperre eine Folie aus geradkettigem Polyethylen geringer Dichte (linear low density polyethylene: LLDPE). In einer Ausführungsform, bei der ein Probengefäß für den zu untersuchenden Boden vorgesehen ist, kann die Dampfsperre auch als LLDPE-Beutel oder Liner ausgebildet sein, der mit dem Bodenmaterial gefüllt wird. Typischerweise beträgt die Dicke der LLDPE-Folie zwischen 5 und 50 µm und bevorzugt zwischen 5 und 10 µm. Der als Einmalartikel verwendbare LLDPE-Beutel wirkt demnach nicht nur als Dampfsperre, sondern ermöglicht außerdem, dass das Probengefäß (beispielsweise eine Kammer aus Edelstahl oder einem anderen Oberflächenmaterial, das Fumigantien nicht zurückhält) nach jeder Verwendung sauber bleibt.

Die erfindungsgemäße Nachweisvorrichtung ist besonders robust und preiswert herstellbar und kann auch zusammen mit einer Anreicherungseinheit sehr kompakt ausgebildet sein. Mit der erfindungsgemäßen Vorrichtung lässt sich daher insbesondere ein tragbares Analysegerät realisieren. Für den Einsatz in der Landwirtschaft kann das Analysegerät beispielsweise eine stabartige Sonde umfassen, die zur Probennahme in den Boden eingestochen werden kann.

Es lassen sich auch stationäre Analysegeräte realisieren, welche Sender umfassen, die die gemessenen Werte, vorzugsweise drahtlos zu einer zentralen Datenerfassungsstelle übertragen.

Die Erfindung wird im folgenden unter Bezugnahme auf ein in den beigefügten Zeichnungen dargestelltes Ausführungsbeispiel detaillierter beschrieben.

In den Zeichnungen zeigen:
- Fig. 1: eine schematische Darstellung eines erfindungsgemäßen tragbaren Analysegeräts zum Nachweis von MITC in der Bodenluft;
- Fig. 2: eine schematische Darstellung der Messkammer das Analysegeräts der Figur 1;
- Fig. 3: eine schematische Darstellung zweier masseempfindlicher Sensorelemente, mit selektiver Oberflächenschicht, wie sie in der Messkammer der Figur 2 verwendet werden;
- Fig. 4: ein beispielhaftes Syntheseprinzip für supramolekulare Bausteine für selektive Oberflächenschichten;
- Fig. 5: eine schematisierte Synthesestrategie für Makrozyklen für selektive Oberflächenschichten;
- Fig. 6: ein Beispiel eines als selektive Oberflächenschicht geeigneten Makrozyklus;
- Fig.7-12: Beispiele für zur Herstellung selektiver Oberflächenschichten geeigneter Dendrimere.

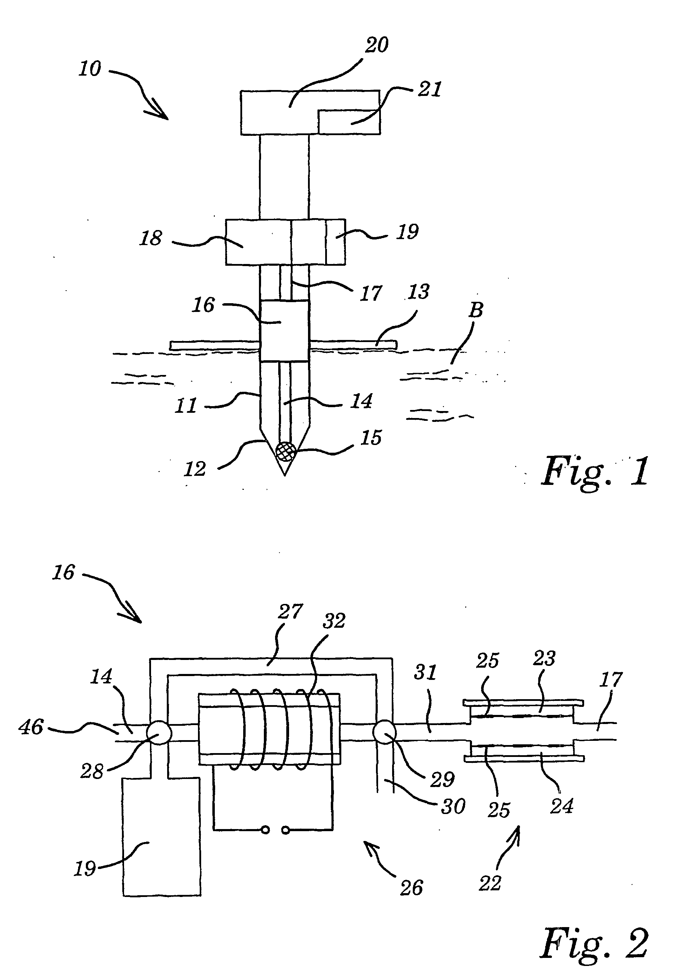

In Fig. 1 ist eine Ausführungsform der erfindungsgemäßen Vorrichtung zum Nachweis von MITC in Luftproben dargestellt. Die erfindungsgemäße Vorrichtung ist als tragbare Stichsonde 10 ausgebildet. Die Sonde 10 weist einen Schaft 11 auf, der zur Aufnahme von Bodenluftproben mit seiner Spitze 12 in den Boden B gesteckt werden kann. Damit die Probennahme stets in einer definierten Tiefe stattfindet, ist am Außenumfang des Schaftes 11 eine kranzförmige Platte 13 vorgesehen, die beim Einstecken des Schaftes in den Boden einen Anschlag bildet. In dem Schaft 11 ist eine Leitung 14 vorgesehen, die in der Spitze 12 in den Außenumfang des Schaftes mündet. Die Mündungsöffnung der Leitung 14 ist durch ein feines Sieb 15 abgedeckt, welches verhindert, dass beim Ansaugen von Bodenluft Erdreich oder andere feste Partikel mitangesaugt werden. Die Leitung 14 führt zu Detektionsmitteln 16, die eine Messkammer umfassen, in welcher ein Schwingquarzwaagen-Array zum Nachweis von MITC angeordnet ist. Eine bevorzugte Ausführungsform der Detektionsmittel 16 wird weiter unten unter Bezugnahme auf die detailliertere Darstellung der Fig. 2 näher erläutert. Eine aus den Detektionsmitteln 16 herausführende Austrittsleitung 17 mündet in Fördermitteln 18, welche beispielsweise als Gebläse oder als Saugpumpe ausgebildet sein können, und welche die Bodenluft über die Leitung 14 durch die Detektionsmittel 16 fördern. Außerdem können eine oder mehrere Kartuschen 19 mit Spül- und/oder Eichgasen vorgesehen sein. Im dargestellten Beispiel sind in einem Griff 20 der Sonde 10 Energieversorgungsmittel, wie beispielsweise Batterien 21, angeordnet, welche die Sonde mit Strom versorgen.

Die Sonde kann geeignete Anzeigeeinrichtungen zur optischen und/oder akustischen Anzeige der Messwerte aufweisen. Ferner können Sende- und Empfangseinrichtungen zur Fernbedienung und Datenübertragung vorgesehen sein. Derartige Einrichtungen sind dem Fachmann geläufig und werden hier nicht näher erläutert.

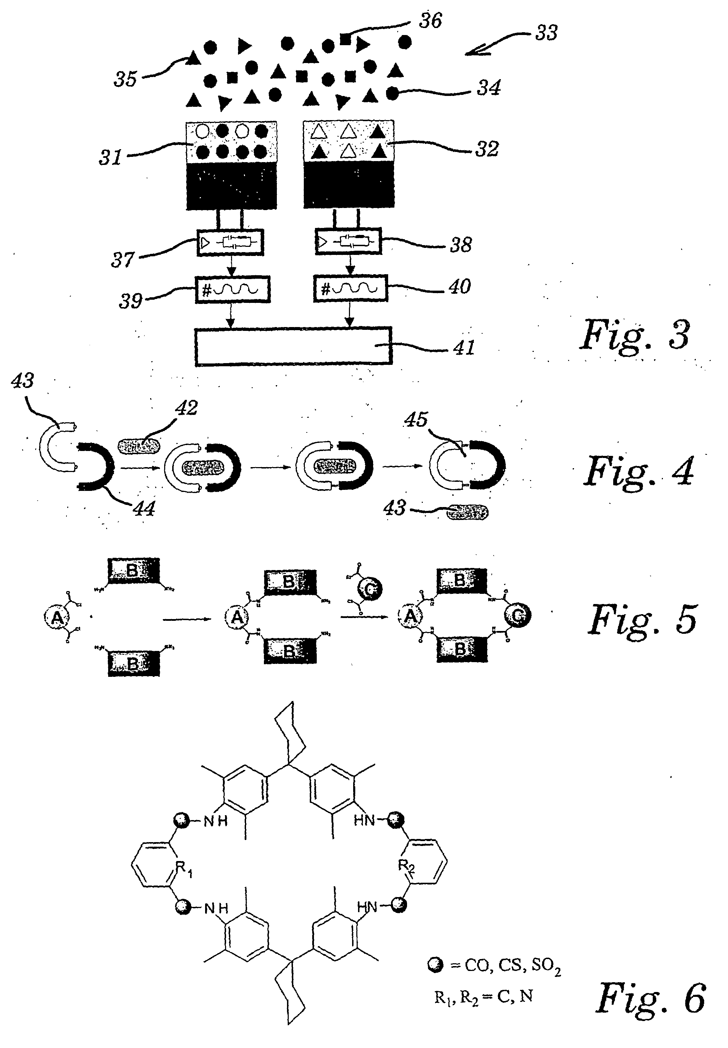

In Fig. 2 ist eine bevorzugte Ausführungsform der Detektionsmittel 16 mit massenempfindlichen Sensoren schematisch dargestellt. Es versteht sich, dass die Detektionsmittel 16 auch dann verwendet werden können, wenn ein (nicht dargestelltes) Probengefäß für die zu untersuchenden Bodenmaterialien benutzt wird. Die Detektionsmittel 16 umfassen eine Messkammer 22, die im dargestellten Beispiel oben und unten von Quarzblättchen 23,24 begrenzt ist. Auf den Quarzblättchen sind mehrere Metallspots 25 aufgedampft, welche die einzelnen Sensoren des Arrays definieren. Beispielsweise über ein Elektrosprühverfahren sind die einzelnen Sensoren mit selektiven Oberflächenschichten beschichtet. Bevorzugt weisen einige der Beschichtungen eine besonders hohe Selektivität für die nachzuweisenden Fumigantien auf. Ein oder mehrere Sensoren können auch mit einem Material beschichtet sein, das für die nachzuweisenden Fumigantien unempfindlich, aber für die in der Luft enthaltende Feuchtigkeit besonders empfindlich ist. Ein bevorzugtes feuchtigskeitsempfindliches Material ist beispielsweise Poly-Vinyl-Pyrrolidon (PPy). Ein oder mehrere Sensoren können auch unbeschichtet bzw. mit einer besonders inerten Beschichtung versehen sein, die weder für Feuchtigkeit, noch für andere Bestandteile des Bodengases sensitiv ist. Derartige Sensoren eignen sich dann insbesondere zur Überwachung einer etwaigen Trift der Auswerteelektronik, wie sie beispielsweise aufgrund von Temperaturschwankungen auftreten kann. Eine detaillierter Darstellung eines derartigen Sensorsystems, insbesondere eine Beschreibung vorteilhafter Messmodi bei der Konzenträtionsbestimmung von Gasen, findet sich in Boeker et al. *Sensors and Actuators B70 (2000), 37-42.* Der Aufbau der einzelnen Sensorelemente wird weiter unten im Zusammenhang mit der Darstellung der Fig. 3 detaillierter erläutert.

In dem in Fig. 2 dargestellten Beispiel ist vor der Messkammer 22 eine Anreicherungseinheit 26 angeordnet. Wenn beispielsweise eine reine Bestimmung der Bodenfeuchtigkeit durchgeführt werden soll oder wenn das nachzuweisende MITC in höheren Konzentrationen vorliegen, kann die Anreicherungseinheit auch über einen Bypass 27 umgangen werden kann. Die Steuerung der Strömungswege erfolgt über geeignete Ventileinrichtungen 28,29, die von einer (hier nicht dargestellten) Kontrolleinheit automatisch gesteuert werden. Für eine Messung mit vorgeschalteter Anreicherung wird zunächst Bodenluft über die Leitung 14 durch die Anreicherungseinheit 26 und die Leitung 30 zu der (in Fig. 2 nicht dargestellten) Pumpe 18 gefördert. Die Verbindungsleitung 31 zu der Messkammer 22 ist geschlossen. In der Anreicherungseinheit 26 kann beispielsweise Kieselgel oder TENAX als Ad- bzw. Absorptionsmittel angeordnet sein. Nach einer bestimmten Anreicherungszeit wird die Leitung 30 geschlossen und die Leitung 31 zur Messkammer 22 geöffnet. Über eine Heizung 32 wird das sorbierte Material thermisch desorbiert und mit Hilfe der Förderpumpe und/oder eines in der Kartusche 19 befindlichen Spülgases aus der Anreichungseinheit 26 in die Messkammer 22 überführt. Bei bestimmten Ausführungsformen der Erfindung ist eine Dampfsperre 46, beispielsweise eine LLDPE-Folie, stromaufwärts von der Messkammer 22 angeordent.

Das Messprinzip einer Schwingquarzwaage, welche erfindungsgemäß zum Nachweis der Fumigantien verwendet wird, ist dem Fachmann aus anderen Bereichen der Analytik bekannt und wird daher unter Bezugnahme auf die Darstellung der Fig. 3 nur kurz erläutert. Jedes einzelne Element des Sensorarrays (zwei dieser Elemente sind in Fig. 3 gezeigt) weist eine selektive Oberflächenschicht 31,32 auf, die für die einzelnen Bestandteile der Bodenluftprobe 33 (symbolisch dargestellt als Kreise 34, Dreiecke 35 oder Rechtecke 36) unterschiedlich empfindlich sind. Beispielsweise ist die Schicht 31 speziell für die als Kreise 34 symbolisierten Luftbestandteile empfindlich, während die Schicht 32 speziell für die als Dreiecke 35 dargestellten Luftbestandteile empfindlich ist. Eine Änderung der Massenbelegung der einzelnen Sensorelemente durch Ad- oder Absorption von Bestandteilen der Luftprobe werden durch Änderung der Resonanzfrequenz einer schematisch dargestellten Oszillatorschaltung 37, 38 über Frequenzzähler 39,40 ausgewertet. Die einzelnen Sensorelemente sind in der Realität jedoch nie hundertprozentig selektiv. So wird beispielweise bei einer realen Messung das Sensorelement 31 auch in gewissem Umfang auf die Luftbestandteile 35 und 36 ansprechen. Daher werden die von den einzelnen Sensorelementen 31,32 gelieferten Messergebnisse von einer nachgeschalteten elektronischen Signalauswertung 41 erfasst, die mittels an sich bekannter chemometrischer Methoden die Konzentrationen der einzelnen Bestandteile in der Bodenluftprobe 33 ermittelt.

Es wurde überraschend gefunden, dass sich zum Nachweis von MITC in Luftproben insbesondere supramolekulare Systeme als selektive Beschichtungen 31,32 eignen. Dabei sind sogenannte Makrozyklen und Dendrimere wegen ihrer Monodispersivität attraktiv, denn sie gestatten den Bau reversibler, schnell ansprechender und regenierbarer Gassensoren. Ferner ermöglichen sie durch ihre mannigfaltig gestaltbaren Hohlräume, die spezifisch an den Raumbedarf des nachzuweisenden Analyten angepasst werden können, eine große Gestaltungsvielfalt und -freiheit. Durch Ausnutzung der Wirt-Gast-Wechselwirkung, zu der auch individuell ausgebildete Wasserstoffbrücken beitragen, oder durch Ausnutzung einer spezifischen Donor-Akzeptor-Wechselwirkung können individuell maßgeschneiderte Wirtsysteme synthetisiert werden. Supramolekulare Wirtssysteme gestatten dabei in besonderem Maße eine optimale Anpassung an den jeweiligen Gast, da beispielsweise Dendrimere eine hohe Toleranz für verschiedenartige funktionelle Gruppen aufweisen.

In Fig. 4 ist beispielhaft das Syntheseprinzip von supramolekularen, nanometergroßen Bausteinen dargestellt, wobei ein Gastmolekül 42 als Template beim Ringschluß zweier supramolekularer Bausteine 43,44 zum Wirtmolekül 45 dient. Auf diese Weise ist eine gezielte Synthese attraktiver Wirte 45 möglich, deren Eignung als substratselektive Schichtsysteme für die hier interessierenden Fumigantien mit Hilfe der erfindungsgemäßen Vorrichtung erprobt und optimiert werden kann.

In Fig. 5 ist beispielhaft eine Synthesestrategie für Makrozyklen dargestellt, welche sich ebenfalls als selektive Oberflächenschicht eignen. Als besonders vorteilhaft erweist sich die hochflexible Synthesestrategie zu Herstellung der Makrozyclen. Sowohl die Seitenteile A,C des späteren Makrozyklus als auch die Spacer B können unabhängig voneinander variiert werden. Somit kann gezielt eine vorher festgelegte Art und Stärke der Wirt-Gast-Wechselwirkung eingestellt werden. Das bedeutet, dass diese Wirtmoleküle ohne großen Aufwand an ihr späteres Aufgabengebiet als sensoraktive Schicht angepaßt werden können.

Es lassen sich sowohl thermodynamische als auch kinetische Parameter der Einlagerungsprozesse für die Adsorption und Desorption spezieller Gastmoleküle bestimmen. Die daraus gewonnene Information kann zur Optimierung bzw. zur Feinunterscheidung artgewandter Gastmoleküle herangezogen werden, so dass sich die Selektivität und die Empfindlichkeit der sensoraktiven Schichten der erfindungsgemäßen Vorrichtung für den Nachweis und die Unterscheidung artverwandter Carbonylverbindungen erheblich verbessern lässt. Die Chemie der Makrozyklen und Catenane sei insbesondere auf die Pionierarbeiten von Vögtle et. al. *Angew. Chem., 104, 1628-1631, (1992); Angew. Chem. Int. Ed. Engl., 31, 1619-1622,* (*1992*) verwiesen. Synthesstrategien finden sich beispielsweise auch in Ottens-Hildebrandt et. al *J. Chem. Soc., Chem. Commun., 777-779, (1995).*

Als besonders geeignete sensoraktive Schicht erweist sich dabei eine durch Amidgruppen zu Wasserstoffbrückenbindung fähige und präorganisierte Wirtstruktur. In Fig. 6 ist beispielhaft der Lactam-Makrozyklus als ringförmiges Molekül dargestellt, das diese Anforderungen erfüllt. Der Ring besitzt vier potentielle Koordinationszentren in Form von Amid-, Thioamid- oder Sulfonamidgruppen.

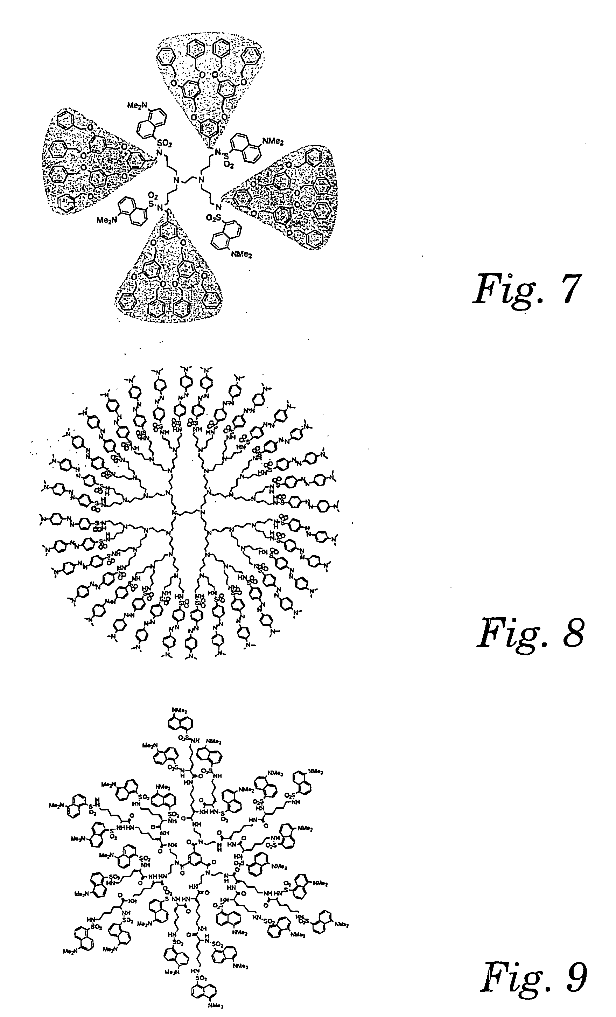

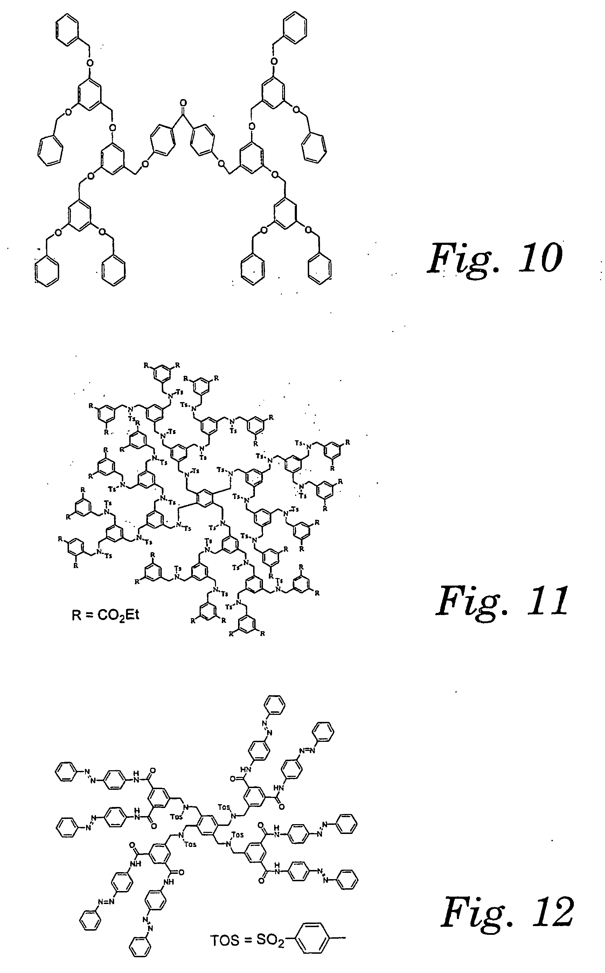

Bevorzugte Dendrimere zur Herstellung selektiver Oberflächenschichten, die sich (zum Nachweis von MITC eignen, sind schließlich in den Fig. 7 bis 12 dargestellt.

### Beispiel:

Eine Vorrichtung zum Nachweis von Methyl-iso-thio-cyanat (MITC) in Luftproben wurde hergestellt durch Beschichten von sechs einzelnen Schwingquarzwaagen mit einer Oberflächenschicht eines als J1 bezeichneten Lactam-Amid-Makrozyklus (die Schwingquarzwaagen wurden mit entsprechender, kommerziell erhältlicher Elektronik von der Firma HKR Sensorsysteme GmbH, München, Deutschland bezogen). Die Betriebs- bzw. Schwingungsfrequenz lag im Bereich von etwa 10 MHz.

### Herstellung von J1:

J1, ein Lactam-Amidmakrozyklus mit der Formel 31'-tert-butyl-5', 19 ' ,25', 37',40 ', 42 ', 45', 47 '-octamethyl-8 ' ,16 ' ,28' ,34'-tetraoxodispiro[cyclohexane-1,2'-[7',17' ,27',35' ,]tetraaza[10']oxyheptacyclo[34.2.2.23', 6'.218', 21'.223' 26'. 111', 15' .129', 33']-heptatetracontal[3',5',11',13',15' (41'),18' ,20', 23', 25', 29', 31', 3.3' (46'), 36', 38', 39', 42', 44']octa-decaen-20', 1"-cyclohexan], wurde wie folgt hergestellt.

Eine Lösung 3-Phenoxyacetyldichlorid in 100 ml Dichlormethan (absolut) wurde in einem gut wärmegetrockneten und mit Argon gespülten Apparat über einen Zeitraum von 5 Stunden bei Raumtemperatur unter kontinuierlichem Rühren in eine Lösung von 10,00 g (32,30 mMol) 1,1-bis(4'-Ammo-3',5'-dimethylphenyl)cyclohexan und 2,2 ml Triethylamin in 50 ml Dichlormethan (absolut) eingetropft. Das resultierende Gemisch wurde über Nacht gerührt. Anschließend wurde das Lösungsmittel bei Unterdruck in einem Rotationsverdampfer entfernt.

Der Rückstand wurde mit einer Silicasäule chromatographisch gereinigt. Man erhielt 1,4 g (37 %) eines farblosen Feststoffs (Schmelzpunkt Mp = 149-151 °C). 1,20 g (1,50 mMol) davon wurden zusammen mit 0,4 ml Triethylamin in 250 ml Dichlormethan aufgelöst. Entsprechend wurden 0,39 g (1,50 mMol) 5-tert.-Butylisophthalsäurechlorid in 250 ml Dichlormethan aufgelöst. Beide Lösungen wurden gleichzeitig in einem Zeitraum von 8 Stunden zu 1 1 des gleichen Lösungsmittels (Dichlormethan) bei Raumtemperatur zugegeben. Anschließend wurde das Reaktionsgemisch für weitere 2 Tage gerührt, woraufhin das Lösungsmittel bei Unterdruck entfernt wurde. Man erhielt 0,047 g (32 %) eines farblosen Feststoffes nach chromatographischer Reinigung auf Silica (Schmelzpunkt Mp > 260 °C). Der farblose Feststoff wurde durch Maldi-MS mit m/z = 191,2 [M⁺] 1.014,2 [M⁺ + Na], 1.030,2 [M⁺ + K] als J1 mit der oben angegebenen Zusammensetzung und Struktur identifiziert.

### Herstellung der Oberflächenschicht:

Die J1-Obenflächenschichten wurden durch Elektrosprühen einer J1-Lösung durch die Kapillare einer entsprechend dimensionierten Spritze auf die oberste Elektrode jeder einzelnen Schwingquarzwaage (QMB) nacheinander aufgetragen, wobei zwischen der Kapillare und der obersten Elektrode jeder QMB ein Hochspannung von etwa 5 kV angelegt wurde. Der Beschichtungsprozess wurde in situ überwacht. Die resultierende Dicke der Beschichtung wurde standardisiert, indem man das Elektrosprühen beendete, sobald die Schwingungsfrequenz um 5 kHz reduziert war.

### Ergebnisse:

Definierte MITC-Konzentrationen wurden durch Einperlen eines gleichmäßigen und definierten Stroms aus reinem Stickstoff in eine MITC-Schmelze erzeugt. Anschließend wurde der Strom mit einer wirksamen Kühlvorrichtung, die auf einer konstanten Temperatur von -12 °C gehalten wurde, abgekühlt. Die so erhaltene definierte Sättigungskonzentration von MITC in Stickstoff wurde durch Mischen mit einem definiert einstellbaren Strom reinen Stickstoffs weiterverdünnt, so dass man Ströme mit unterschiedlichen Konzentrationen von MITC in Stickstoff erzeugen konnte.

Ströme mit MITC-Konzentrationen in Stickstoff von 10, 25, 50, 100 oder 200 ppm wurden bei einer Durchflussrate von 22 ml pro Minute durch Ansaugen durch eine Messkammer geleitet, in der das *QmB-Ar*ray angeordnet war. Die QMBs und die Messkammer wurden auf einer konstanten Temperatur von 35 °C gehalten. Es wurde eine Verringerung der Schwingungsfrequenzen der einzelnen QMBs beobachtet, die proportional zur MITC-Konzentration war, nämlich 2,5, 7,0, 14,0, 24,0 bzw. 37,5 Hz.

Mit der erfindungsgemäßen Vorrichtung können demnach MITC-Konzentrationen im Bereich von wenigen ppm genau und reproduzierbar gemessen werden.

## Patentansprüche

1. Verfahren zum Nachweis von Methyl-iso-thio-cyanat in Bodenluftproben, wobei man
eine Bodenluftprobe ansaugt,
die angesaugte Bodenluftprobe mit wenigstens einem massenempfindlichen Sensor in Kontakt bringt, der wenigstens eine Schwingquarzwaage umfasst, die mit einer für die Aufnahme von Methyl-iso-thio-cyanat selektiven Oberflächenschicht, die Makrozyklen und/oder Dendrimere umfaßt, beschichtet ist,
Massenänderungen des Sensors in Form von elektrischen Signalen detektiert; und
die elektrischen Signale auswertet.

2. Verfahren gemäß Anspruch 2, **dadurch gekennzeichnet, dass** man mehrere Sensoren verwendet, die mit unterschiedlichen selektiven Schichten beschichtet sind.

3. Verfahren gemäß einem der Ansprüche 1 oder 2, **dadurch gekennzeichnet, dass** man das nachzuweisende Methyl-iso-thio-cyanat anreichert, bevor man es mit dem massenempfindlichen Sensor in Kontakt bringt.

4. Verfahren gemäß einem der Ansprüche 1 bis 3, **dadurch gekennzeichnet, dass** man außerdem den Feuchtigkeitsgehalt der Bodenluftprobe bestimmt.

5. Verfahren gemäß einem der Ansprüche 1 bis 4, **dadurch gekennzeichnet, dass** man der Bodenluftprobe Feuchtigkeit entzieht, bevor man sie mit dem Senor in Kontakt bringt.

6. Verfahren zur Bekämpfung von Phytopathogenen, **dadurch gekennzeichnet, dass** man Erdboden mit einer wirksamen Menge eines Methyl-iso-thio-cyanat freisetzenden Bodenbegasungsmittels behandelt und anschließend vor einer Wiedereinsaat bzw. Wiedeinpflanzung von Nutz- und Kulturpflanzen Restmengen an freigesetztem Methyl-iso-thio-cyanat mit dem Verfahren nach einem der Ansprüche 1 bis 5 nachweist.

7. Vorrichtung zum Nachweis von Methyl-iso-thio-cyanat in Bodenluftproben, insbesondere zur Durchführung des Verfahrens nach einem der Ansprüche 1 bis 5, mit
Probennahmemitteln (11,18) zur Aufnahme einer Bodenluftprobe,
Detektionsmitteln (16), welche bei Kontakt mit der Bodenluftprobe elektrische Signale erzeugen, die von der Konzentration des nachzuweisenden Methyl-iso-thio-cyanats in der Bodenluftprobe abhängen,
Auswertemitteln (41), welche aus den von den Detektionsmitteln gelieferten elektrischen Signalen die Konzentration des in der Bodenluftprobe enthaltenen Methyl-iso-thio-cyanats berechnen,
wobei
die Detektionsmittel (16) wenigstens einen massenempfindlichen Sensor (33) umfassen, der wenigstens eine Schwingquarzwaage umfasst, die mit einer für das nachzuweisende Methyl-iso-thio-cyanat selektiven Oberflächenschicht (31,32), die Makrozyklen und/oder Dendrimere umfaßt, beschichtet ist.

8. Vorrichtung gemäß Anspruch 7, **dadurch gekennzeichnet, dass** die Probennahmemittel eine Anreicherungseinheit (26) für das nachzuweisende Methyl-iso-thio-cyanat umfassen.

9. Vorrichtung gemäß einem der Ansprüche 7 oder 8, **dadurch gekennzeichnet, dass** die Probennahmemittel Mittel (46) zum Entfernen von Feuchtigkeit aus der Bodenluftprobe aufweisen.

10. Tragbares Analysegerät, umfassend eine Vorrichtung nach einem der Ansprüche 7 bis 9, sowie eine Einrichtung zur Anzeige und/oder Fernübertragung der von den Auswertemitteln (41) berechneten Konzentrationen an Methyl-iso-thio-cyanat.

## Claims

1. A method for detecting methyl isothiocyanate in soil air samples, wherein
a soil air sample is drawn in,
the drawn-in soil air sample is brought into contact with at least one mass-sensitive sensor which comprises at least one quartz microbalance, said quartz microbalance being coated with a surface layer which is selective for incorporation of methyl isothiocyanate and which comprises macrocycles and/or dendrimers,
mass changes of the sensor are detected in the form of electrical signals, and
the electrical signals are evaluated.

2. A method as claimed in claim 1, wherein a plurality of sensors coated with different selective layers are used.

3. A method as claimed in any of claims 1 or 2, wherein the methyl isothiocyanate to be detected is concentrated before making contact with the mass-sensitive sensor.

4. A method as claimed in any of claims 1 to 3, wherein the moisture content of the soil air sample is additionally determined.

5. A method as claimed in any of claims 1 to 4, wherein moisture is removed from the soil air sample before it is brought into contact with the sonsor.

6. A method of controlling phytopathogens, which comprises treating soil with an effective amount of a methyl isothiocyanate releasing soil fumigant and subsequently, prior to new sowings or plantings of useful plants or crop plants, detecting residual amounts of released methyl isothiocyanate with the method as claimed in any of claims 1 to 5.

7. A device for detecting methyl isothiocyanate in soil air samples, in particular for carrying out the method as claimed in any of claims 1 to 5, with
sampling means (11, 18) for sampling soil air,
detection means (16), which, upon contact with the soil air sample, generate electrical signals which depend on the concentration of the methyl isothiocyanate to be detected in the soil air sample,
evaluation means (41) which calculate the concentration of the methyl isothiocyanate present in the soil air sample on the basis of the electrical signals provided by the detection means,
wherein said detection means (16) comprise at least one mass-sensitive sensor (33) which comprises at least one quartz microbalance, said quartz microbalance being coated with a surface layer (31, 32) which is selective for the methyl isothiocyanate to be detected and which comprises macrocycles and/or dendrimers.

8. A device as claimed in claim 7, wherein the sampling means comprise a concentration unit (26) for the fumigants to be detected.

9. A device as claimed in any of claims 7 or 8, wherein the sampling means comprise means (46) for removing moisture from said soil air samples.

10. A portable analyzer, comprising a device as claimed in any of claims 7 to 9 and a unit for displaying and/or transmitting the fumigant concentration calculated by the evaluation means (41).

## Revendications

1. Procédé pour déceler la présence d'isothiocyanate de méthyle dans des échantillons d'air au sol, dans lequel
on aspire un échantillon d'air au sol,
on met en contact l'échantillon d'air au sol aspiré avec au moins un capteur sensible à la masse qui comprend au moins une balance à cristal de quartz oscillateur, qui est revêtu d'une couche superficielle sélective pour l'absorption d'isothiocyanate de méthyle, qui comprend des macrocycles et/ou des dendrimères,
on détecte les changements de masse du capteur sous forme de signaux électriques ; et
on analyse les signaux électriques.

2. Procédé selon la revendication 1, **caractérisé en ce qu'**on utilise plusieurs capteurs qui sont revêtus de couches sélectives différentes.

3. Procédé selon l'une quelconque des revendications 1 ou 2, **caractérisé en ce qu'**on enrichit l'isothiocyanate de méthyle à déceler avant de le mettre en contact avec le capteur sensible à la masse.

4. Procédé selon l'une quelconque des revendications 1 à 3, **caractérisé en ce qu'**on détermine en outre la teneur en humidité de l'échantillon d'air au sol.

5. Procédé selon l'une quelconque des revendications 1 à 4, **caractérisé en ce qu'**on élimine l'humidité de l'échantillon d'air au sol avant de le mettre en contact avec le capteur.

6. Procédé pour combattre les phytopathogènes, **caractérisé en ce qu'**on traite le sol avec une quantité efficace d'un agent de gazage du sol libérant de l'isothiocyanate de méthyle et **en ce qu'**on détermine ensuite, avant un nouvel ensemencement ou une nouvelle plantation de plantes utiles et de culture, les quantités résiduelles de l'isothiocyanate de méthyle libéré avec le procédé selon l'une quelconque des revendications 1 à 5.

7. Dispositif pour déceler la présence d'isothiocyanate de méthyle dans des échantillons d'air au sol, en particulier pour la mise en oeuvre du procédé selon l'une quelconque des revendications 1 à 5, avec
des moyens de prélèvement d'échantillon (11,18) pour le prélèvement d'un échantillon d'air au sol,
des moyens de détection (16), qui produisent par contact avec l'échantillon d'air au sol des signaux électriques, qui dépendent de la concentration de l'isothiocyanate de méthyle à déceler dans l'échantillon,
des moyens d'analyse (41) qui calculent d'après les signaux électriques délivrés par les moyens de détection, la concentration de l'isothiocyanate de méthyle contenu dans l'échantillon d'air au sol,
dans lequel
les moyens de détection (16) comportent au moins un capteur sensible à la masse (33) qui comporte au moins une balance à cristal de quartz oscillateur, qui est revêtu d'une couche superficielle sélective pour l'isothiocyanate de méthyle à déceler (31, 32), qui comprend des macrocycles et/ou des dendrimères.

8. Dispositif selon la revendication 7, **caractérisé en ce que** les moyens de prélèvement d'échantillon comprennent une unité d'enrichissement (26) pour l'isothiocyanate de méthyle à déceler.

9. Dispositif selon l'une quelconque des revendications 7 ou 8, **caractérisé en ce que** les moyens de prélèvement d'échantillon présentent des moyens (46) pour l'élimination de l'humidité de l'échantillon.

10. Appareil d'analyse portatif, comportant un dispositif selon l'une quelconque des revendications 7 à 9 ainsi qu'un dispositif pour l'indication et/ou la télétransmission des concentrations en isothiocyanate de méthyle calculées par les moyens d'analyse (41).
